Europäisches Patentamt

European Patent Office    (11) Publication number: **0 046 915**

Office européen des brevets    **B1**

(12) **EUROPEAN PATENT SPECIFICATION**

(45) Date of publication of patent specification: **31.10.84**    (51) Int. Cl.³: **C 07 C 7/00, C 12 P 21/00**

(21) Application number: **81106358.5**

(22) Date of filing: **17.08.81**

(54) **A method for the recovery of a peptide-containing compound, and an apparatus for the realization of the method.**

(30) Priority: **02.09.80 SE 8006102**

(43) Date of publication of application:
**10.03.82 Bulletin 82/10**

(45) Publication of the grant of the patent:
**31.10.84 Bulletin 84/44**

(84) Designated Contracting States:
**AT BE CH DE FR GB IT LI LU NL SE**

(56) References cited:
**EP-A-0 028 016**
**DE-A-2 639 129**
**FR-A-2 211 469**
**FR-A-2 215 621**
**FR-A-2 252 351**
**FR-A-2 366 303**
**GB-A-2 023 816**

**MAKROMOL. CHEM., vol. 179, no. 5, May 1978 BASEL (DE) A. CHAABOUNI et al.: "Synthèse de poly(oxyéthylène)s rendus biospécifiques par fixation de stéroides en extrémités de**

(73) Proprietor: **Gambro Lundia AB**
**Box 10101**
**S-220 10 Lund (SE)**

(72) Inventor: **Nylen, Ulf Thomas Gustav**
**Borgarevägen 15**
**S-222 47 Lund (SE)**

(74) Representative: **Boberg, Nils Gunnar Erik**
**Gambro AB Patent Department Box 10101**
**S-220 10 Lund (SE)**

(56) References cited:
**chaînes. Utilisation d'un poly(oxyéthylène) substitué par des groupes estradiol pour extraire l'isomérase delta5-4 oxo-3 stéroide, par partage d'affinité", pages 1135-1144**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Technical field

The present invention relates to a method for the recovery of a peptide-containing compound, wherein a crude extract containing said peptide-containing compound together with possible contaminations is contacted with a solution containing a biospecific ligand attached to a carrier in soluble form so as to form a complex of carrier/ligand/peptide-containing compound, and wherein said peptide-containing compound is liberated from the so formed complex and separated from said solution.

The invention relates moreover to an apparatus for the realization of the above-mentioned method.

Background art

It is known in the art to recover a peptide-containing compound through contacting a solution, containing a biospecific ligand attached to a carrier, so as to form a complex of carrier/ligand/peptide-containing compound, and then liberating and separating said peptide-containing compound from the so formed complex.

For example, according to US Patent 3 850 798 the ligand used shall be attached to a carrier which is a solid substance and which is insoluble in the solution from which the peptide-containing compound is to be recovered. Thus, for example, the carrier with the ligand attached to it may be present in the form of particles which have been collected to a mass in a column through which the solution is made to flow in order to achieve contact between the ligand and the peptide-containing compound so as to form a complex of the carrier, the ligand and the peptide-containing compound.

An improvement over the method according to said US Patent is however obtained if the ligand used is attached to a carrier in dissolved form, such as is disclosed in FR—A—2 252 351 and FR—A—2 211 469 according to which the peptide-containing compound is recovered from a crude extract through precipitating.

It has been found, inter alia, that with such a means in dissolved form a more uniform and better distribution of the said means throughout the solution is achieved, as a result of which a more effective formation of contact between the peptide-containing compound and the attached ligand is produced. The diffusion length between these two substances, for example, will be shorter than if the said means is present in solid form. Furthermore, any steric and/or mechanical hindrances for contact between the two substances will be fewer, which likewise contributes to an increase in the effectiveness of the method, because as a result the peptide-containing compound can be fixed onto the ligand, and later liberated from the same, more readily.

A disadvantage of the method according to said FR—A—2 252 351 and 2 211 469 is, however, that the formed complex of carrier/ligand/peptide-containing compound is obtained in form of a precipitate.

According to the invention it has been found that an improvement of the method according to FR—A—2 252 351 and FR—A—2 211 469 is achieved if the method throughout is performed in a homogeneous phase-system, i.e. an essentially homogeneous liquid-phase system.

One major advantage obtained with such a homogeneous phase-system is that the method is well adapted for a run on a continuous basis which in turn is well suited for large-scale recovery of the concerned peptide-containing compound. Furthermore, while using an essentially homogeneous liquid-phase system, process parameters such as flow conditions are easily controlled, and disturbances inherent to for example clogging solids are completely avoided. Still further, an apparatus for performing a process in a homogeneous phase system can be made less complicated in comparison to an apparatus developed for use in a heterogeneous phase system process.

Disclosure of invention

In accordance with the present invention a method is thus provided for the recovery of a peptide-containing compound, wherein a crude extract containing said peptide-containing compound together with possible contaminations is contacted with a solution containing a biospecific ligand attached to a carrier in soluble form so as to form a complex of carrier/ligand/peptide-containing compound, and wherein said peptide-containing compound is liberated from the so formed complex and separated from said solution. The method in accordance with the invention is characterized in that said possible contaminations are separated from said solution at different stages during the method depending on their respective molecular weights, whereby the method is performed in an essentially homogeneous liquid-phase system.

Preferably said crude extract is subjected to a first membrane filtration before the contact with said solution so as to remove possible contaminations with molecular weights above the molecular weight of the peptide-containing compound, e.g. $5 \times 10^5$ Dalton.

The solution with the complex formed is conveniently subjected to a second membrane filtration before said liberating of the peptide-containing compound, so as to remove remaining contaminations with molecular weights which are smaller than the molecular weight of the complex formed.

2

Examples of membrane filters which can be used in accordance with the invention, are hollow fibers with a cut-off around $5 \times 10^5$ Dalton (D). Membrane filters in forms other than the said hollow fibers may also be used, for example plane or helical membranes.

For the further separation of any compounds of a contaminant type present, the solution which contains the complex formed of carrier, ligand and peptide-containing compound is also subjected to a membrane filtration using a membrane filter of the same type as that described above, but with a cut-off which is preferably somewhat higher than the earlier one. Examples of such membrane filters may be hollow fibers with a cut-off around $10^6$ D. However, this cut-off must be such that the complex formed is retained in the solution.

To increase the degree of separation of these compounds it may be appropriate to use several membrane filters coupled in series, the solution being made to flow in turn through these filters with successively growing separation of the compounds. To compensate for the loss in liquid which occurs as a result, and to maintain at the same time an appropriate pH in the remaining solution, a weak buffer solution is added appropriately to the remaining solution in connection with the series filtration. The term appropriate pH here means a pH which is such that the bond between the ligand and the peptide-containing compound is not broken. Examples of such appropriate pH values may be between 6 and 9.

To liberate the peptide-containing compound from the complex formed, an acid is added appropriately which alters the pH of the solution and thereby breaks the bond. Alternatively this bond may be broken by adding a little salt solution which increases the ionic strength of the solution.

The peptide-containing compound thus liberated can then be separated and recovered by means of a further membrane filtration, using a membrane filter which is adapted so that it lets through the peptide-containing compound liberated, but retains the carrier with the ligand attached to it. This membrane filter too may be of the same type as those mentioned earlier. A suitable cut-off is between $5 \times 10^5$ and $10^6$ D.

To restore the pH of the solution after separation and recovery of the peptide-containing compound, a base is added appropriately which raises the pH of the solution to the neutral point. The solution so adjusted, containing the carrier with ligand attached, may then be used anew.

The choice of ligand is determined by the type of peptide-containing compound which is to be recovered, inasmuch as these two substances must be able to fix onto one another by biospecific affinity.

Examples of ligands usable in accordance with the invention are immunoglobulins, Fc-receptors, certain surface receptors from bacteria with affinity for the Fc-part (e.g. S. aureus protein A), co-factors and certain sugar-containing proteins.

Examples of peptide-containing compounds which can be recovered by the method in accordance with the invention and which consequently can be biospecific affinity fix onto, for example anyone of the examples listed above of usable ligands may be antigens, immunoglobulins, enzymes and lecithins.

Among examples of carriers in accordance with the invention may be mentioned soluble macromolecules with molecular weights above the molecular weight of the peptide-containing compound which is to be recovered. Examples of such macromolecules may be soluble types of sugars, in particular cellulose or dextran. Another example would be a soluble polyacrylamide.

The ligand is attached to the carrier preferably by bonds of a covalent character. Such bonds can be achieved, for example, by periodate activation of the carrier with subsequent coupling to the ligand. The said bonds may be achieved by diimidazole coupling. Various other procedures for achieving such attachments between a carrier and a ligand are well-documented in the available literature on the subject and do not need to be discussed in detail here.

According to the invention an apparatus for the realization of the described method is also provided. The apparatus is characterized by comprising a membrane filter comprising a semipermeable membrane adapted to let lower molecular weight contaminations having a molecular weight lower than the molecular weight (such as below $10^6$ D) of the formed complex of carrier/ligand/peptide-containing compound to pass therethrough so as to separate said lower-molecular weight contaminations from said solution, and a membrane filter comprising a semipermeable membrane adapted to let the liberated peptide-containing compound pass therethrough while retaining present compounds, including the ligand attached to the carrier, having a molecular weight within a specified range, such as $5 \times 10^5$ to $10^6$ D, so as to separate said liberated peptide-containing compound from said solution.

According to a preferred embodiment, the apparatus further comprises a membrane filter comprising a semi-permeable membrane adapted to retain higher molecular contaminations having a molecular weight above the molecular weight of said peptide-containing compound, such as above $5 \times 10^5$ D, so as to prevent said higher molecular weight compounds present in said crude extract from contacting said solution containing the ligand attached to the carrier.

Brief description of drawings

In the following the invention will be further illustrated in connection with a practical realization of the method in accordance with the invention with reference to the enclosed drawing, wherein

Fig. 1 shows schematically how the separate stages in the method in accordance with the invention can be integrated to a continuous process, and

3

Fig. 2 by way of example shows a suitable practical embodiment of a membrane filter for use in the process illustrated in Fig. 1.

Best mode of carrying out the invention

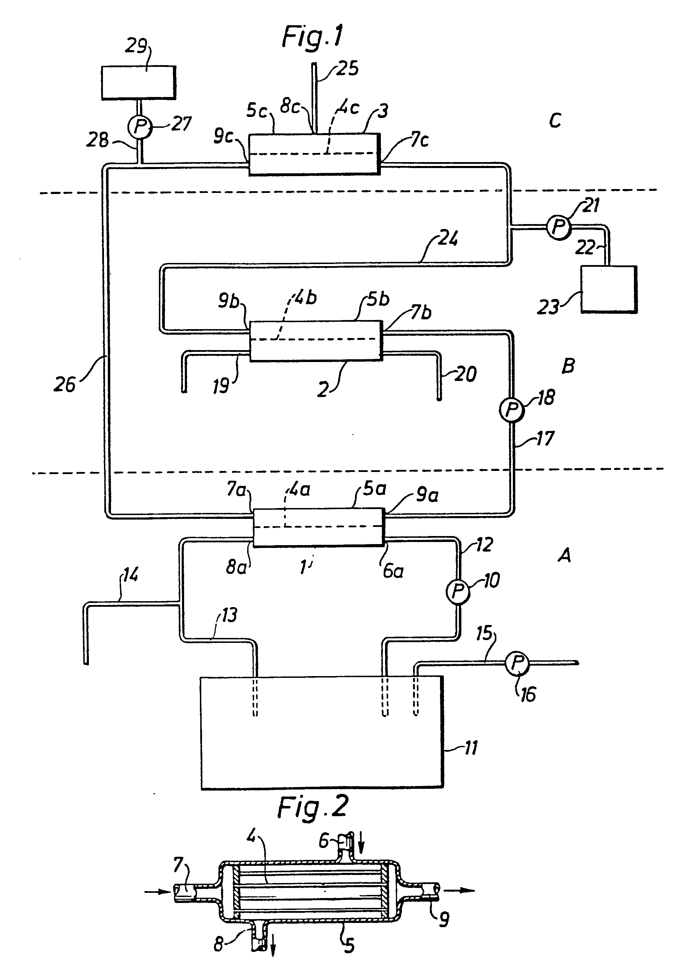

In Fig. 1 letters A, B and C are intended schematically to represent the separate stages in the method according to the invention. Thus letter A designates the contact stage, B designates the stage of the liberation of the peptide-containing compound and C designates the separation and recovery stage.

In everyone of these stages preferably a membrane filter 1, 2 and 3 respectively is used with cut-off depending upon the compound which is to be recovered. A suitable embodiment of such a filter is shown schematically in Fig. 2. Here the filter is designated as a so-called artificial kidney of the hollow-fiber type with hollow fibers 4 enclosed within an outer jacket 5 with inlets 6, 7 and corresponding outlets 8, 9. For a more detailed description of such an artificial kidney, reference is made, for example, to US patent 3 228 877. For corresponding parts of the membrane filters 1—3 the same reference numerals are used here as in Fig. 2, but with the addition of the letters a, b and c respectively.

Stage A

The solution which contains the peptide-containing compound which is to be recovered is pumped with the help of a pump 10 from a culture tank 11 for bacteria, which can produce this compound, to the membrane filter 1 via a line 12 and an inlet 6a. Inside the membrane filter 1 the solution, which is conducted through the space between the hollow fibers 5a and the jacket 5a, will be filtered through the pores in the fiber walls owing to the pressure difference which is maintained between this space and the inner cavities of the fibers. The hollow fibers 4a are adapted so that contaminations of molecular weights above a certain defined limit, e.g. $5 \times 10^5$ D, are retained in the solution outside the fibers, whilst compounds (including the peptide-containing compound which is to be recovered) with molecular weights below this limit pass through the wall pores of the fibers into the inner cavity of the fibers to make contact with the solution which flows through this cavity and which contains the carrier with ligand attached thereto. The retained solution outside the fibers is conducted out of the membrane filter 1 through the outlet 8a and is returned to the culture tank 11 via a line 13 after drawing off the bacteria slop through a withdrawal line 14. Fresh culture medium is supplied via a line 15 with the help of a pump 16.

Stage B

From the stage A the solution with the complex of carrier, ligand and peptide-containing compound formed is pumped with the help of a pump 18 from the membrane filter 1 to the membrane filter 2 via a line 17 connected to the outlet 9a on the membrane filter 1 and the inlet 7b on the membrane filter 2. Inside the membrane filter 2 the solution is filtered through the pores in the hollow fibers 4b so as to separate any contaminations which may have remained in the solution. The hollow fibers 4b are adapted so that contaminations of molecular weights below a certain defined limit, e.g. $10^6$ D, pass through the wall pores out of the inner cavity of the fibers, whilst compounds (including the complex formed) with molecular weights above this limit are retained in the solution inside the cavity of the fibers.

To compensate for the loss in liquid, which occurs owing to the filtration in the membrane filter 2, and in order to maintain an appropriate pH in the remaining solution, a weak buffer solution is added appropriately through a line 19 in connection with the filtration. The filtrate formed is discharged via a line 20.

After this filtration of the solution with the complex formed, a means for altering the pH is added, for example an acid, so as to lower the pH of the solution and thereby to break the bond between the ligand and the peptide-containing compound so as to liberate the peptide-containing compound from the complex. This addition may be done, for example, with the help of a pump 21 in a line 22 connected to a source 23 of acid and a line 24 which is connected to the outlet 9b on the membrane filter 2 and the inlet 7c on the membrane filter 3 and which is arranged so that it conducts solution from the membrane filter 2 to the membrane filter 3.

Stage C

The solution with the peptide-containing compound thus freed is conducted into the membrane filter 3, into the cavities of the hollow fibers 4c, via the said inlet 7c, for the separation and recovery of the liberated peptide-containing compound via a line 25 in connection with the outlet 8c on the membrane filter 3. The hollow fibers 4c are adapted so that compounds (including the peptide-containing compound liberated) with molecular weights below a defined limit, e.g. $5 \times 10^5$ or $10^6$ D, pass through the wall pores of the fibers, 4c, whilst compounds (including the carrier and the ligand attached to it) with molecular weights above the same limit are retained in the solution inside the cavity of the fibers.

From the membrane filter 3 the solution with the carrier and the ligand attached thereto is returned to the membrane filter 1 via a line 26 in connection with the outlet 9c on the membrane filter 3 and the inlet 7a on the membrane filter 1 for re-use in stage A.

To restore the pH of the solution, a base (possibly together with carrier/ligand) is added appropriately with the help of a pump 27 in a line 28 connected to a source 29 of base and the line 26.

In the following the invention will be described in more detail with the help of concrete embodiments relating to the method as well as to the auxiliary means for the realization of the same.

Example 1

Recovery of protein A with dextran as the carrier for the ligand which is constituted of an immunoglobulin.

Material

IgG, purified by protein A affinity chromatography on solid phase attached to Dextran-2000 $(M=2\times10^6 D)$ (4.2 IgG per molecule dextran).

Protein A labelled by $I^{125}$

IgG from affinity chromatography on solid phase, labelled by $I^{131}$.

Citric acid (pA), potassium dihydrogenphosphate (pA), dipotassium hydrogenphosphate (pA).

Membrane filter in the form of hollow fibers with a specified cut-off of $10^6$ D.

Experiment I: To 500 ml of 0.1 M phosphate buffer at pH 7.3 are added 100 ml of a solution which contains IgG attached to Dextran-2000. Radioactive protein A is added in deficit. The solution is mixed for 1 minute and subsequently pumped with a flow rate of approximately 150 ml/min through the cavities in the membrane fibers and back to the mixing vessel. The filtrate formed fills successively the space outside the fibers. For the sake of clarity it should be mentioned that the hollow fibers are enclosed within an outer jacket in the same manner as, for example, in a so-called artificial kidney of the hollow-fiber type. The filtrate is drawn off at the top at the rate as it is formed. A pressure difference of 10—40 cm $H_2O$ is maintained, at which dialyzate is formed at a rate of approximately 40 ml/min. Samples are taken at different stages of the concentration process from the dialyzate as well as from the solution used. The solution is diluted to 2l and the concentration process continues during the sample taking.

Blue dextran of a molecular weight $2\times10^6$ D has been tested earlier and was found to pass the membrane so that the concentration in the filtrate will be 2.5% of the concentration in the solution used under similar conditions.

The measurement of the respective samples was carried out in a gamma counter: $(spA^x I^{125})$.

The result of the measurements is shown in Table I.

TABLE I

| Sample[xx] | | Protein A measured value cpm/2 ml | | % Protein A which have passed through the membrane in relation to the liquid volume |
|---|---|---|---|---|
| | No. | Main solution | Filtrate | |
| | 1 | 31,000 | 4,200 | 13.5 |
| | 2 | 47,000 | 4,700 | 10.0 |
| | 3[x] | 31,092 | 2,854 | 9.2 |
| | 4 | 41,102 | 2,879 | 7.0 |

[x]dilution of the remaining solution

[xx]samples No. 1, 2, 3 and 4 were drawn after the formation of 100, 400, 800 and 1,300 ml filtrate respectively.

Owing to the somewhat long standstill in connection with the start of the experiment (sample 1), a little too much protein A had passed through the membrane initially. The figure of 10%, however, may be regarded as fairly adequate. The end value of 7% (sample 4) is probably due to the fact that the quantity of free IgG has diminished in the main solution owing to the dialysis process.

Since only 2.5% dextran should have passed the membrane, if all the protein A added had become attached to the dextran, there is good reason for assuming that 7.5% derive from protein A which is attached to free IgG. Since protein A which is attached to IgG in free from passes almost unhindered through the membrane (retention approximately 20%; based on the result of experiment II),

## 0 046 915

it thus appears from this experiment that the binding capacity for free IgG constitutes less than 10% of the binding capacity for dextran-attached IgG.

Dialysate and main solution are put together and the pH is adjusted to 3 (measured with pH-paper) by means of 1 M citric acid. The solution is mixed and a new concentration process is started. Volume $\simeq$2.3l.

The following measuring results were obtained:

TABLE II

| Sample[x] | Protein A measured value, cpm/2 ml | | % Protein A liberated |
|---|---|---|---|
| No. | Main solution | Dialyzate | |
| 5 | 14,800 | 12,380 | 83.6 |
| 6 | 15,900 | 13,410 | 84.3 |
| 7 | 18,110 | 14,400 | 79.5 |

[x]Samples No. 5, 6 and 7 taken after formation of 400, 1,000 and 1,400 ml dialyzate respectively.

Table II shows that the protein A attached is liberated and can readily be filtered out at pH=3.

Experiment II: The procedure is the same as in experiment I, but beside radioactive protein A $I^{125}$, which is added last, 10 mg IgG labelled by $I^{131}$ are also added.

The solution is immediately diluted to 2l and mixed for 1 minute pH=7.3.

To determine the interference between $I^{125}$ and $I^{131}$, a measurement is carried out first on the pure preparation with the two "windows" which will be used later. "Window" A (0—40) and "window" B (50—500). The following measured values were obtained:

| Window | Measured value, cpm/2 ml | |
|---|---|---|
| | $I^{125}$ | $I^{131}$ |
| A | 16,350 | 308 |
| B | 40 | 2,325 |

Thus in window B, within the accuracy of measurement, exclusively $I^{131}$ was measured, whilst the measuring result in window A is a sum of $I^{131}$ and $I^{125}$. The measuring results given below (Table III) have been compensated so that measured values are indicated for each isotope separately.

TABLE III

| Sample[x] | Protein A measured value cpm/2 ml | | IgG measured value cpm/2 ml | |
|---|---|---|---|---|
| No. | Main solution | Dialyzate | Main solution | Dialyzate |
| 8 | 15,018 | 5,974(25.1%) | 17,904 | 11,778(66%) |
| 9 | 17,868 | 7,250(41%) | 17,928 | 16,067(89%) |
| 10 | 23,558 | 7,570(32%) | 19,600 | 17,328(88%) |
| 11 | 33,823 | 7,713(22%) | 20,500 | 17,304(84%) |

[x]Samples No. 8, 9, 10 and 11 taken after formation of 200, 800, 1,200 and 1,600 ml dialyzate respectively.

The per cent figures in parentheses in the above Table III indicate the measure of protein A and the quantity of IgG respectively which have passed through the membrane. Table III makes evident, therefore, that free IgG easily passes the membrane, and also that free IgG with attached protein A passes readily. The experiment equally shows that 10 mg IgG which have been added together with the IgG already present, constitute a good 30% of the binding capacity, that is to say 10 mg IgG which have been added constitute approximately 20% of the bonding capacity and the dextran-attached IgG

6

70%. 50 mg IgG or a protein A binding capacity of approximately 15 g exists therefore in added dextran.

The experiment also makes evident that this short filtration has led to the "contamination" IgG being reduced to approximately 50% in relation to the protein A.

The pH is adjusted to 3 (measured with pH paper) by means of 1 M citric acid (volume approx. 2.3l) after the dialyzate formed earlier has been mixed again with the main solution. The solution is concentrated as before. The following result was obtained:

TABLE IV

| Sample[x] | Protein A measured value cpm/2 ml | | IgG measured value cpm/2 ml | |
|---|---|---|---|---|
| No. | Main solution | Dialyzate | Main solution | Dialyzate |
| 12 | 11,906 | 11,863(100%) | 12,933 | 6,828(52%) |
| 13 | 11,978 | 10,507(88%) | 12,867 | 7,055(55%) |
| 14 | 13,106 | 9,602(73%) | 14,828 | 6,682(45%) |
| 15 | 16,719 | 11,424(69%) | 20,730 | 7,252(35%) |

[x]Sample 12, 13, 14 and 15 taken after formation of 400, 800, 1,200 and 1,800 ml dialyzate respectively.

The per cent figures in parentheses in Table IV indicate the measure of the quantity of protein A and IgG respectively which pass through the membrane. Table IV makes evident, therefore that protein A passes well through the membrane, whilst IgG for some reason is strongly retarded at the pH, as distinguished from its behaviour at pH=7.3 (Table III). Presumably this is due to the pH having become somewhat too low, giving rise to (aggregation) denaturation of IgG, which means then that the protein cannot be dialyzed. Such a denaturation (unspec. aggregation) may even be thought to affect the solubility of protein A through occlusion.

The above-mentioned experiments suggest that this system can be used for the purification of protein A. To obtain satisfactory affinity-chromatographic purification with the membrane and carrier used in the example it is necessary, however, that the filtration and concentration stages are repeated a number of times. In an industrial process it would be necessary therefore, in order to simplify and to accelerate the process, if the above-mentioned membrane is used, to employ a somewhat larger carrier molecule, dextran or cellulose of a molecular weight around $5 \times 10^6$ D. Alternatively, a membrane with somewhat smaller pores can be used, that is to say a memrbane which has a cut-off (approximately 90% retention) of $7 \times 10^5$ D instead of that used of approximately $10^6$ D.

Example II

Coupling of IgG to dextran according to the method with periodate activation.

Materials

Dextran T 2000 (Pharmacia Fine Chemicals), sodium metaperiodate (Merck) and sodium borohydride (Merck).

2 gram of dextran T 2000 are dissolved in 50 ml of distilled water. 10 ml of newly prepared 0.1 M sodium periodate solution (in water) are added and the whole mixture is allowed to stand with stirring for 20 minutes at room temperature. Subsequently the mixture is dialyzed against 1.0 mM sodium acetate buffer, pH 4.4, for 6 hours at room temperature with change of buffer every hour. The pH in the mixture is increased to 9.3 by addition of 0.2 M sodium carbonate buffer, pH 9.5, 310 ml of IgG solution (obtained by dialysis against 0.01 M carbonate buffer, pH 9.5, during 6 hours at room temperature and subsequent concentration and storage in the cold) are added, and the whole mixture is stirred for 2 hours. The IgG solution had also been dialyzed against 0.01 M sodium carbonate buffer, pH 0.3, during 2 hours. 66 mg of sodium borohydride dissolved in 8 ml of distilled water are added. The mixture is allowed to stand in the refrige chest for 2 hours and is filtered subsequently through a membrane with a cut-off of $1 \times 10^6$ D. A calculation of the yield showed that 4.2 molecules of IgG had been coupled per molecule of dextran T 2000. The overall yield was as high as approximately 75%.

Industrial applicability

The method in accordance with the invention is applicable to the recovery of a protein in free form. It can be utilized, for example, in continuous processes, where the recovery of the protein takes place in that a solution containing the protein is brought into contact with an immunoglobulin attached

# 0 046 915

to a carrier so as to fix the protein by biospecific affinity to the immunoglobulin, and where the protein so fixed is afterwards separated from the immunoglobulin in order to obtain the protein in free form. Protein A, in particular, can be recovered by bringing the said solution into contact with IgG which has been attached to soluble dextran.

## Claims

1. A method for the recovery of a peptide-containing compound, wherein a crude extract containing said peptide-containing compound together with possible contaminations is contacted with a solution containing a biospecific ligand attached to a carrier in soluble form so as to form a complex of carrier/ligand/peptide-containing compound, and wherein said peptide-containing compound is liberated from the so formed complex and separated from said solution, characterized in that said possible contaminations are separated from said solution at different stages during the method depending on their respective molecular weights, whereby the method is performed in an essentially homogeneous liquid-phase system.

2. A method in accordance with claim 1, characterized in that said crude extract is subjected to a first membrane filtration before the contact with said solution so as to remove possible contaminations with molecular weights above the molecular weight of the peptide-containing compound, e.g. $5 \times 10^5$ Dalton.

3. A method in accordance with claim 1 or 2, characterized in that the solution with the complex formed is subjected to a second membrane filtration before said liberating of the peptide-containing compound, so as to remove remaining contaminations with molecular weights which are smaller than the molecular weight of the complex formed.

4. A method in accordance with any one of the preceding claims, characterized in that the peptide-containing compound is liberated through an alteration of the pH, e.g. through addition of an acid so as to break the bond between the ligand and the peptide-containing compound.

5. A method in accordance with any one of claims 1—3, characterized in that the peptide-containing compound is liberated through the addition of a salt solution to increase the ionic strength of the solution.

6. A method in accordance with claim 4 or 5, characterized in that the peptide-containing compound liberated is separated and recovered by means of a third membrane filtration with use of a membrane filter (3) which is adapted so that it lets through the peptide-containing compound, but retains the carrier with the ligand attached to it, e.g. a membrane filter with a cut-off between $5 \times 10^5$ and $10^6$ Dalton.

7. A method in accordance with claim 6, characterized in that the solution with the carrier and the ligand attached to it is re-used, possibly after restoration of the pH of the said solution.

8. A method in accordance with claim 3, characterized in that a weak buffer solution is added in connection with the second membrane filtration so as to maintain the pH of the solution around the neutral point.

9. An apparatus for the recovery of a peptide-containing compound according to the method as defined by claim 1, characterized by comprising a membrane filter (5b) comprising a semipermeable membrane (4b) adapted to let lower molecular weight contaminations having a molecular weight lower than the molecular weight (such as below $10^6$ Dalton) of the formed complex of carrier/ligand/peptide-containing compound to pass therethrough so as to separate said lower-molecular weight contaminations from said solution, and a membrane filter (5c) comprising a semipermeable membrane (4c) adapted to let the liberated peptide-containing compound pass therethrough while retaining present compounds, including the ligand attached to the carrier, having a molecular weight within a specified range, such as $5 \times 10^5$ to $10^6$ Dalton, so as to separate said liberated peptide-containing compound from said solution.

10. An apparatus in accordance with claim 9, characterized by further comprising a membrane filter (5a) comprising a semipermeable membrane (4a) adapted to retain higher molecular contaminations having a molecular weight above the molecular weight of said peptide-containing compound, such as above $5 \times 10^5$ Dalton, so as to prevent said higher molecular weight compounds present in said crude extract from contacting said solution containing the ligand attached to the carrier.

## Patentansprüche

1. Verfahren zur Gewinnung einer peptidhaltigen Verbindung, bei dem ein Rohextrakt, das diese peptidhaltige Verbindung zusammen mit möglichen Verunreinigungen enthält, mit einer Lösung behandelt wird, die einen an einen Träger gebundenen biospezifischen Liganden in löslicher Form enthält, so daß sich ein Komplex von Träger/Ligand/peptidhaltiger Verbindung bildet, und die peptidhaltige Verbindung aus dem so gebildeten Komplex freigesetzt und von der Lösung abgetrennt wird, dadurch gekennzeichnet, daß die möglichen Verunreinigungen von der Lösung in verschiedenen Stufen während des Verfahrens je nach ihren betreffenden Molekulargewichten abgetrennt werden, wobei das Verfahren in einem System mit im wesentlichen homogener flüssiger Phase durchgeführt wird.

8

2. Verfahren nach Anspruch 1, dadurch gekennzeichnet, daß das Rohextrakt vor der Berührung mit der Lösung einer ersten Membranfiltration unterzogen wird, um so mögliche Verunreinigungen mit Molekulargewichten oberhalb des Molekulargewichts der peptidhaltigen Verbindung, wie $5 \times 10^5$ D, zu entfernen.

3. Verfahren nach Anspruch 1 oder 2, dadurch gekennzeichnet, daß die Lösung mit dem gebildeten Komplex vor der Freisetzung der peptidhaltigen Verbindung einer zweiten Membranfiltration unterzogen wird, um so restliche Verunreinigungen mit Molekulargewichten, die kleiner als das Molekulargewicht des gebildeten Komplexes sind, zu entfernen.

4. Verfahren nach einem der vorausgehenden Ansprüche, dadurch gekennzeichnet, daß die peptidhaltige Verbindung durch eine Änderung des pH-Wertes freigesetzt wird, wie durch Zugabe einer Säure, um die Bindung zwischen dem Liganden und der peptidhaltigen Verbindung aufzubrechen.

5. Verfahren nach einem der Ansprüche 1 bis 3, dadurch gekennzeichnet, daß die peptidhaltige Verbindung durch die Zugabe einer Salzlösung zur Erhöhung der Ionenstärke der Lösung freigesetzt wird.

6. Verfahren nach Anspruch 4 oder 5, dadurch gekennzeichnet, daß die freigesetzte peptidhaltige Verbindung mit Hilfe einer dritten Membranfiltration unter Verwendung eines Membranfilters (3), das die peptidhaltige Verbindung durchläßt, aber den Träger mit dem daran gebundenen Liganden zurückhält, wie mit einem Membranfilter mit einer Ausschlußgrenze zwischen $5 \times 10^5$ und $10^6$ D, abgetrennt und gewonnen wird.

7. Verfahren nach Anspruch 6, dadurch gekennzeichnet, daß die Lösung mit dem Träger und dem daran gebundenen Liganden wiederverwendet wird, gegebenenfalls nach Wiederherstellung des pH-Wertes dieser Lösung.

8. Verfahren nach Anspruch 3, dadurch gekennzeichnet, daß eine schwache Pufferlösung in Verbindung mit der zweiten Membranfiltration zugegeben wird, um so den pH-Wert der Lösung um den Neutralpunkt herum zu halten.

9. Apparatur zur Gewinnung einer peptidhaltigen Verbindung nach dem Verfahren gemäß Anspruch 1, gekennzeichnet durch ein Membranfilter (5b) mit einer semipermeablen Membran (4b), die so ausgebildet ist, daß sie niedrigermolekulare Verunreinigungen mit einem Molekulargewicht unter dem Molekulargewicht (wie unter $10^6$ D) des gebildeten Komplexes von Träger/Ligand/peptidhaltiger Verbindung hindurchgehen läßt, um diese niedrigermolekulare Verunreinigungen von der Lösung abzutrennen, und ein Membranfilter (5c) mit einer semipermeablen Membran (4c), die so ausgebildet ist, daß sie die freigesetzte peptidhaltige Verbindung hindurchgehen läßt, während sie vorhandene Verbindungen, einschließlich des an den Träger gebundenen Liganden, mit einem Molekulargewicht in einem spezifizierten Bereich, wie $5 \times 10^5$ bis $10^6$ D, zurückhält, um so die freigesetzte peptidhaltige Verbindung von der Lösung abzutrennen.

10. Apparatur nach Anspruch 9, weiter gekennzeichnet durch ein Membranfilter (5a) mit einer semipermeablen Membran (4a), die so ausgebildet ist, daß sie höhermolekulare Verunreinigungen mit einem Molekuargewicht oberhalb des Molekulargewichts der peptidhaltigen Verbindung, wie oberhalb $5 \times 10^5$ D, zurückhält, um so zu verhindern, daß diese in dem Rohextrakt vorhandenen höhermolekularen Verbindungen in Berührung mit der Lösung, die den an den Träger gebundenen Liganden enthält, kommen.

**Revendications**

1. Procédé pour la récupération d'un composé contenant un peptide dans lequel un extrait brut contenant le dit composé contenant un peptide avec des contaminations éventuelles est mis en contact avec une solution contenant un ligand biospécifique attaché à un support sous une forme soluble de façon à former un complexe support/ligand/composé contenant un peptide, et dans lequel ledit composé contenant un peptide est libéré du complexe ainsi formé et séparé de ladite solution, caractérisé en ce que lesdites contaminations éventuelles sont séparées de ladite solution en des stades différents pendant le procédé selon leurs poids moléculaires respectifs, le procédé étant ainsi effectué dans un système en phase liquide essentiellement homogène.

2. Procédé selon la revendication 1, caractérisé en ce que ledit extrait brut est soumis à une première filtration sur membrane avant le contact avec ladite solution pour éliminer les contaminations éventuelles ayant des poids moléculaires supérieurs au poids moléculaire du composé contenant un peptide, par exemple $5 \times 10^5$ daltons.

3. Procédé selon la revendication 1 ou 2, caractérisé en ce que la solution, avec le complexe formé, est soumise à une seconde filtration sur membrane avant ladite libération du composé contenant un peptide, de façon à éliminer les contaminations restantes ayant des poids moléculaires qui sont inférieurs au poids moléculaire du complexe formé.

4. Procédé selon l'une quelconque des revendications précédentes, caractérisé en ce que le composé contenant un peptide est libéré par modification du pH, par exemple par addition d'un acide de façon à rompre la liaison entre le ligand et le composé contenant un peptide.

5. Procédé selon l'une quelconque des revendications 1 à 3, caractérisé en ce que le composé contenant un peptide est libéré par addition d'une solution d'un sel pour accroître la force ionique de la solution.

6. Procédé selon la revendication 4 ou 5, caractérisé en ce que le composé contenant un peptide libéré est séparé et récupéré au moyen d'une troisième filtration sur membrane qui utilise un filtre à membrane (3) qui est conçu de façon à laisser passer le composé contenant un peptide mais à retenir le support avec le ligand qui lui est attaché, par example un filtre à membrane ayant une limite de séparation entre $5 \times 10^5$ et $10^6$ daltons.

7. Procédé selon la revendication 6, caractérisé en ce que la solution, avec le support et le ligand qui lui est fixé, est réutilisée, éventuellement après rétablissement du pH de ladite solution.

8. Procédé selon la revendication 3, caractérisé en ce qu'une solution tampon faible est ajoutée en association avec la seconde filtration sur membrane de façon à maintenir le pH de la solution au voisinage du point neutre.

9. Appareil pour le récupération d'un composé contenant un peptide selon le procédé tel que défini par la revendication 1, caractérisé en ce qu'il comprend un filtre à membrane (5b) comprenant une membrane semi-perméable (4b) conçue pour laisser passer les contaminations de poids moléculaires inférieurs ayant un poids moléculaire inférieur au poids moléculaire (par exemple inférieurs à $10^6$ daltons) du complexe formé de support/ligand/composé contenant un peptide de façon à séparer lesdites contaminations de poids moléculaires inférieur de ladite solution, et un filtre à membrane (5c) comprenant une membrane semi-perméable (4c) conçue pour laisser passer le composé contenant un peptide libéré en retenant les composes présents, y compris le ligand attaché au support, ayant un poids moléculaire compris dans une gamme déterminée telle que $5 \times 10^5$ à $10^6$ daltons de façon à séparer ledit composé contenant un peptide libéré d'avec ladite solution.

10. Appareil selon la revendication 9, caractérisé en ce qu'il comprend de plus un filtre à membrane (5a) comprenant une membrane semiperméable (4a) conçue pour retenir les contaminations de poids moléculaires supérieurs ayant un poids moléculaire supérieur au poids moléculaire dudit composé contenant un peptide, par exemple supérieur à $5 \times 10^5$ daltons, de façon à empêcher que lesdits composés de poids moléculaires supérieurs presents dans ledit extrait brut viennent en contact avec ladite solution contenant le ligand attaché au support.

*Fig.1*

*Fig.2*